# EUROPÄISCHE PATENTSCHRIFT

(11) **EP 0 503 012 B1**
(45) Veröffentlichungstag und Bekanntmachung des Hinweises auf die Patenterteilung: **26.06.1996**
(21) Anmeldenummer: 91914844.5
(22) Anmeldetag: 03.09.1991
(51) Int. Cl.: A61M 5/32, B21G 1/08, B23K 26/00

(54) **HOHLNADEL ZUR MEDIZINISCHEN VERWENDUNG UND VERFAHREN ZUR HERSTELLUNG DERARTIGER HOHLNADELN**
HOLLOW NEEDLE FOR MEDICAL USE, AND PROCESS FOR THE MANUFACTURE OF SUCH NEEDLES
CANULE POUR UTILISATION MEDICALE ET PROCEDE POUR LA FABRICATION DE TELLES CANULES

(30) Priorität: 28.09.1990 CH 3131/90
(43) Veröffentlichungstag der Anmeldung: 16.09.1992
(73) Patentinhaber: SULZER INNOTEC AG, CH-8401 Winterthur (CH)
(72) Erfinder: BITTNER, Vladimir, CH-3007 Bern (CH); DEKUMBIS, Roger, CH-8050 Zürich-Oerlikon (CH)
(74) Vertreter: Hammer, Bruno, Dr.
(86) Internationale Anmeldenummer: CH9100189
(87) Internationale Veröffentlichungsnummer: WO9205816

(56) Entgegenhaltungen:
- EP-A- 0 301 246
- CH-A- 253 593
- DE-A- 3 344 709
- FR-A- 2 222 170
- US-A- 2 748 769

## Beschreibung

Die Erfindung bezieht sich auf eine Hohlnadel mit Kanal, der im Bereich der Nadelspitze eine Öffnung aufweist. Derartige Nadeln werden in der Medizin verwendet, beispielsweise zum Injizieren und zum Aspirieren, wie Punktieren von Flüssigkeiten. Weiter bezieht sich die Erfindung auf ein Verfahren zum Herstellen derartiger Hohlnadeln.

Die Spitzen herkömmlicher Hohlnadeln, wie Injektionskanülen, sind, vereinfacht gesagt, scharf geschliffene Stahlröhrchen. Beispielswiese durch einen Schrägschliff entsteht eine scharfe äussere Schliffkante, welche eine glatte Injektionstechnik erlaubt. Dabei entsteht aber gleichzeitig auch auf der Kanülen-Innenseite, d.h. an der Kante des Kanals, dem inneren Auge, eine ebenso scharfe, in Einstichrichtung betrachtet, hintere Schliffschneide, was dazu führen kann, dass Haut und darunterliegendes Gewebe ausgestanzt werden und in das Innere der Injektionskanüle gelangen.

Dies kann zur Folge haben, dass der Kanal der Nadel verstopft, dass empfindliche und schlecht regenerierende Gewebestrukturen, wie Nervenfasern verletzt werden und/oder, dass infizierte, ausgestanzte Hautstückchen in tiefere bzw. andere Gewebestrukturen oder Gewebeschichten verschleppt werden, was zu Spritzenabszessen führen kann.

Der Mangel der herkömmlichen Nadelspitze ist bekannt. So hat H.I. Quincke schon 1891 eine nach ihm benannte verbesserte Spinalnadel beschrieben, die bis heute zur Lumbalpunktion gebraucht wird. Die Quincke-Spinalnadel hat einen exakt angepassten Mandrin (Obturator/Füllkörper/Einlegestab), welcher das innere Lumen der Injektionskanüle vor eindringendem ausgestanztem Material, z.B. Gewebe oder Membranmaterial, schützt. Nach erfolgter Punktion wird der Mandrin entfernt, die Liquorflüssigkeit kann nun problemlos aufgezogen werden.

A. Bier erkannte bereits bei der erstmals im Selbstversuch erfolgten Spinalanästhesie im Jahr 1898, dass der Verlust von Liquorflüssigkeit durch den Punktionsdefekt in der Dura/Arachnoidea (auch Meningen, oder die Hirn- und Rückenmarkshaut genannt) zu lästigem postspinalem Kopfschmerz führen kann. Die Häufigkeit des postspinalen Kopfschmerzes ist neben Alter, Geschlecht und der individuellen Prädisposition vom Durchmesser der Nadel, sowie der Art der Nadelspitze abhängig. R. Hoyt hat im Jahr 1922 auf die Bedeutung von Nadeldurchmessern hingewiesen und hat die Zwei-Nadel-Technik beschrieben, eine dickere Nadel wird als Introducer (Einführhilfe) zuerst eingeführt, durch diese wird eine feinere Nadel bis in den Subarachnoidalraum vorgeschoben. Der Punktionsdefekt der Dura/Arachnoidea bleibt dadurch begrenzt, die Kopfschmerzrate wird reduziert. H.M. Greene hat 1926 eine rundgeschliffene Nadelspitze beschrieben, welche die Fasern der Dura und Arachnoidea schonend auseinanderdrängt, aber nicht durchtrennt, wie dies mit der herkömmlichen Quincke-Nadel mit der scharfen äusseren Schliffkante der Fall ist. Eine Weiterentwicklung der "atraumatischen Spinalnadel" wurde 1951 durch Hart und Whitacre beschrieben: eine Nadel mit geschlossener bleistiftspitzartiger Nadelspitze mit einer seitlichen Öffnung in dieser Spitze. Diese Nadel wurde 1987 durch Sprotte noch weiter verbessert, die seitliche Öffnung wurde vergrössert, um einen Düseneffekt bei Einspritzen des Lokalanästhetikums in den Liquor zu verhindern.

E.B. Tuohy hat 1944 die Methode der kontinuierlichen Spinalanästhesie beschrieben. Zur Punktion verwendete er eine sogenannte Huber-Kanüle, eine Injektionskanüle mit abgebogener Spitze. Die Biegung ist so gestaltet, dass die Schliffebene der Nadelspitze parallel zur Kanüle geführt werden kann. Das hat zur Folge, dass die hintere Schliffschneide der Kanülenspitze hinter der Biegung versteckt werden kann, die Ausstanzgefahr wird dadurch reduziert, jedoch nicht verhindert. R.S.Wagner Jr. verbesserte 1957 die Huber-Kanüle, indem er an der Nadelspitze den Biegungsradius verkleinerte und dadurch eine bessere Führung der Schliffebene parallel zur Kanüle erreichte und die Länge des Schliffauges verkürzte. Damit wurde die exaktere Plazierung der Nadelspitze erreicht. P.A. Cheng modifizierte 1958 die abgebogene Nadelspitze nochmals, indem er die vordere Schliffschneide abstumpfte und die Schliffebene leicht angewinkelt zur Kanülenebene führte. Die hintere Schliffschneide bleibt jedoch bei allen oben beschriebenen Ausführungsarten scharf. Deshalb wird zur Verminderung der Ausstanzgefahr während der Punktion, in die Nadel ein angepasster Mandrin eingeführt, der die hintere Schliffschneide, d.h. die Kante der Öffnung, verdeckt.

H. Haindel und H. Müller haben 1989 eine stanzarme Kanüle mit konkavem Spitzenschliff beschrieben (Biomed. Technik, 34 (1989), 79-84), deren hintere Kante der Öffnung des Kanals mit einer speziellen Glasperlen-Strahlbehandlung abgestumpft ist. Die Perlstrahlbehandlung kann zu Rückständen im Kanal der Hohlnadel führen, welche kaum entfernt werden können. Die Perlstrahlbehandlung, welche kaum eine gezielte und selektive Behandlung der hinteren, inneren Schliffschneide erlaubt, kann dazu führen, dass nur eine ungenügende innere Rundung der Kante der Öffnung erzielt wird.

Die Verwendung dieser Kanüle wird zur Schonung der Silikonmembran von Portsystemen (Aufnahme des Spritzgutes aus membranverschlossenen Behältern, die Membran wird mit der Nadel durchstochen / der Behälter kann implantiert sein) empfohlen Die Anwendung bei anderen Punktionentechniken (Anästhesie, Neurochirurgie, Radiologie) in Geweben, welche weniger konsistent als Silikongummi sind, werden Gewebeteile ausgestanzt. Dies ist insbesondere der Fall, bei der Punktion von Nervengewebe, welches über wenig Eigenelastizität und Konsistenz verfügt.

In CH 253.593 ist ebenfalls eine Hohlnadel beschrieben bei welcher der Rand in der Gegend gegenüber der Nadelspitze abgerundet ist. In diesem Dokument finden sich keine Hinweise, wie die Rundung erzeugt wird.

Unter der Bezeichnung Sprotte-Nadel ist eine Hohlraumnadel mit bleistiftspitzförmiger Spitze bekannt, bei der die Kanülenöffnung im zylindrischen Teil seitlich angebracht ist. Die Öffnung des Kanals ist bei der Sprotte-Nadel relativ weit von der Spitze entfernt. Zudem wird die Sprotte-Nadel mit einem Mandrin verwendet, um das Eindringen von Gewebspartikel in die Kanülenöffnung zu verhindern. Die Verwendung bei Punktionen, die während der Punktion einen freien Flüssigkeitsrückfluss durch die Nadel gewährleisten müssen, ist deshalb kaum möglich.

Die seitlich angebrachte Öffnung des Kanals, das Kanülenauge, stellt eine Schwachstelle der Kanüle dar und das Verbiegen der Kanüle oder gar der Bruch der Hohlnadel bei Knochenkontakt sind zu befürchten.

DE-A-3 344 709 beschreibt ein Verfahren zum Entgraten von metallischen Werkstückrändern mit einer Lasereinrichtung. Mit dem Laserstrahl werden Grate auch an schwierig zugänglichen Lagen von Werkstücken durch Abdampfen oder Abschmelzen entfernt.

Aus medizinischer Sicht betrachtet, ist das Ausstanzen von Gewebepartikeln für den Patient mit einem vermehrten Gewebetrauma verbunden und deshalb unerwünscht. Dies ist inbesondere dann der Fall, wenn die Kanüle in schlecht regenierbare Gewebe, wie Nerven (Leitungsanästhesien) oder Hirn (Neurochirurgie), eingeführt werden soll.

Das Verschleppen von Hautstanzpartikeln in andere Gewebeschichten kann bei Injektion von Steroiden, welche die Immunabwehr beeinträchtigen, zu gefürchteten Spritzenabszessen führen. Das Aufsuchen und Plazieren von Kanülen in Gewebsstrukturen, in denen die Lage der Hohlnadel anhand frei rückfliessender Flüssigkeit verifiziert und Kontrolliert wird (Blut, Liquor, Lymphe, Synovialflüssigkeit, Galle, Ergüsse, Eiteransammlungen etc.), wird erschwert, wenn das Lumen/der Kanal durch Gewebeteile oder sonstwie verengt oder gar verstopft wird. Eine mehrfache Punktion (Einstich) ist die notwendige Folge, häufig verbunden mit Gewebstraumen und vermehrter Schmerzbelastung für den Patient.

Die Erfindung, wie sie in den Ansprüchen gekennzeichnet ist, schafft eine verbesserte Hohlnadel, die auch bei Mehrfachanwendung stets ein offenes Lumen behält, d.h. bei welcher der Kanal beispielsweise nicht durch Späne von Membranen und/oder aus dem Einstichkanal ausgestanzte Gewebereste verstopft oder verunreinigt wird. Die abhängigen Ansprüche 2 bis 7 beziehen sich auf vorteilhafte Ausbildungsformen der Hohlnadel.

Die zu Beginn scharfe und schmelzzurundende Kante nimmt in der Flüssigphase, bei der Laserbehandlung, aufgrund der Oberflächenspannung eine rundum vorzüglich gerundete, nicht schneidende, stumpfe Form an, in der die Kante des Kanals bzw. der Kanüle wieder erstarrt.

Das Verfahren zur Herstellung der Hohlnadel, wie es durch die Merkmale des unabhängigen Anspruchs 8 gekennzeichnet ist, ermöglicht es, die Kante des Kanals, durch exaktes Dosieren der Energie des Laserstrahls bzw. des wirksamen Teils des Laserstrahls, im gewünschten Masse aufzuschmelzen, wobei die Kante nicht nur aussen, sondern auch im Innern des Kanals gerundet wird. Die abhängigen Ansprüche 9 und 10 beziehen sich auf vorteilhafte Weiterbildungen des Verfahrens.

Das Schmelzrunden mit Laserstrahlen erlaubt es, Hohlnadeln aus verschiedensten Werkstoffen und verschiedenster Abmessungen zu bearbeiten. Insbesondere ist es mit diesem Verfahren möglich, die Kanten der Kanäle von Hohlnadeln mit einem Aussendurchmesser von weniger als 0,5 mm, beispielsweise im Aussendurchmesser-Bereich von 0,5 bis 0,2 mm, schmelzzurunden. Ebenso ist es möglich, die Kanten der Öffnungen der Kanäle von Mehrkanal-Hohlnadeln bei Mehrfachkanülen durch Schmelzen zu runden.

Als Laserquellen können Gas- oder Festkörperlaser, beispielsweise CO₂- oder Nd-YAG-Laser, geeignet sein, die gepulst betrieben werden. Die zu bearbeitenden Hohlnadeln können dabei mit einer Transportvorrichtung im Pulstakt des Lasers zu dessen Arbeitsbereich befördert werden. Dabei ist dafür zu sorgen, dass sich die schmelzzurundende Kante jeweils während des Pulses im richtigen Brennfleck bzw. dem Arbeitspunkt des Laserstrahls befindet.

Es ist aber auch denkbar, den Laser kontinuierlich zu betreiben und die Verweilzeit der schmelzzurundenden Bereiche der Kante des Kanals so zu halten, dass die gewünschte Rundung der Kante erfolgt.

In der Regel genügen bei gepulsten Lasern für Hohlnadeln mit einem Aussendurchmesser von etwa einem mm oder weniger Pulse mit einer Gesamtpulsenergie im Bereich von etwa 0,02 bis 20 Joule. Dabei kann der Durchmesser des Arbeitsbereichs bzw. des Arbeitsflecks des Laserstrahls etwa dreimal so gross wie der Aussendurchmesser der Hohlnadel gewählt werden.

Mit Vorteil erfolgt das Schmelzrunden der Kanten unter einem Schutzgas, wie z.B. Stickstoff, Argon oder Helium. Die Beschaffenheit des schmelzgerundeten Teils der Kante des Kanals der Hohlnadeln, kann mit dem Schutzgas, das gleichzeitig als Kühlgas dient, beeinflusst werden.

Die Hohlnadel für medizinische Verwendung weist einen Kanal auf, der meistens eine elliptische oder ellipsenähnliche Öffnung aufweist. Die Kante dieser Öffnung ist in dem, von der Spitze der Hohlnadel betrachtet, konvergierenden Teil schmelzgerundet. Das Verfahren zum Schmelzrunden der Kante verwendet einen gepulst oder kontinuierlich arbeitenden Laser, in dessen Arbeitsbereich der schmelzzurundenen Teil der Kante liegt.

Im folgenden wird die Erfindung anhand der in den schematischen Zeichnungen dargestellten Beispiele näher erläutert:
Es zeigen:
- Fig . 1A und 1B:: In Front- und Seitenansicht die Nadelspitze einer Hohlnadel mit gebogener Spitze und gebogenem Kanal;
- Fig. 2A und 2B:: in Front- und Seitenansicht die Spitze einer Hohlnadel mit Facettenschliff;
- Fig. 3A und 3B:: in Front- und Seitenansicht eine Hohlnadel mit geradem Kanal und schräg geschliffener Nadelspitze;
- Fig. 4a und 4b:: perspektivisch und in einem Seitenschnitt die Anordnung von Laserstrahl und Hohlnadelspitze beim Schmelzrunden.

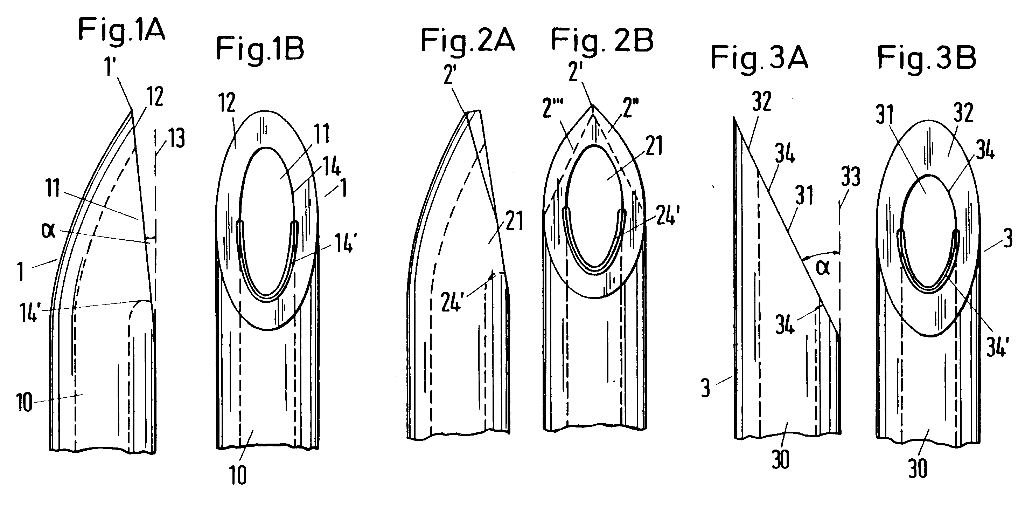

Die in Fig. 1A und Fig.1B stark vergrössert gezeigte Spitze 1' der Hohlnadel 1 bzw. Kanüle 1, ist gebogen. Auch der Kanal 10 ist im Bereich der elliptischen Öffnung 11 gebogen. Die Öffnung 11 mündet in die angeschliffene Ebene 12, die mit der Verlängerung 13 der Mantellinie und damit auch mit der Achse der Hohlnadel 1, einen Winkel α im Bereich von etwa 6° bildet. Anstelle der angeschliffenen Ebene 12 könnt auch eine konkave oder konvexe Fläche vorhanden sein. Die Kante 14 der der Öffnung 11 des Kanals 10 ist im von der Spitze 1' der Hohlnadel 1 betrachtet hinteren, konvergierenden Teil 14' (übertrieben vergrössert dargestellt) schmelzgerundet. Natürlich ist es auch ohne weiteres möglich, weitere Bereiche oder die ganze Kante 14 der Öffnung 11 des Kanals 10 schmelzzurunden. Der äussere Biegeradius der Spitze 1' von derartigen Nadeln 1 liegt mit Vorteil im Bereich des doppelten Aussendurchmessers der Hohlnadel 1. Der Aussendurchmesser der Hohlnadel 1 kann im ultradünnen Bereich von wenigen Zehntelmillimetern, z.B. 0.3 mm aber auch im Dicknadelbereich von über einem Millimeter liegen.

Die Spitze 2' der Hohlnadel 2 von Fig. 2A und 2B ist Bereich der Spitze 2' mit den beiden facettenartigen Schliffebenen 2'' und 2''' ausgebildet. Dieser Facettenschliff verbessert die Sticheigenschaften der Hohlnadel 2. Im übrigen ist die Hohlnadel 2 ebenfalls gebogen und und ähnlich ausgebildet, wie die Spitze 1' der Hohlnadel 1 von Fig. 1A und Fig. 1B. Insbesondere weist die Öffnung 21 ebenfalls einen schmelzgerundeten Bereich 24 auf.

Die Spitze 3' der geraden Hohlnadel 3 weist einen Schrägschliff 32 auf, in welchen die Öffnung 31 des Kanals 30 mündet. Auch hier ist die elliptische Kante 34 der Öffnung 31 des Kanals 30 im von der Spitze 3' der Hohlnadel 3 betrachtet hinteren, konvergierenden Teil 34' (übertrieben vergrössert dargestellt) schmelzgerundet. Der Winkel α der Verlängerung 33 zur Ebene des Schrägschliffs liegt beispielsweise im Bereich von etwa 30°. Diese Ausbildungsform der Hohlnadel ist z.B. für ultradünne Kanülen mit einem Aussendurchmesser von 0,5 mm und weniger geeignet.

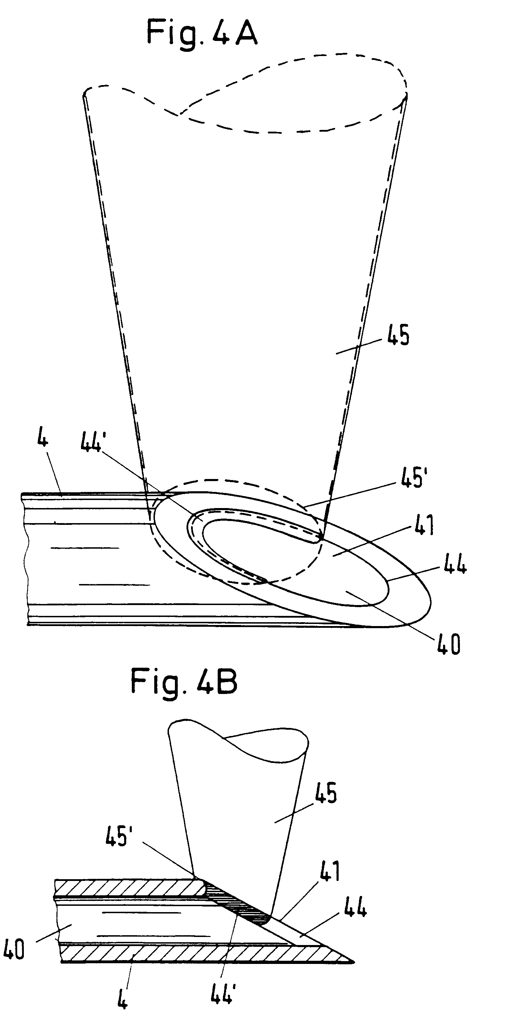

Fig. 4A und Fig. 4B schliesslich zeigen den Laserstrahl 45 und seinen Wirkungsbereich 45' mit der Hohlnadel 4 im Moment des Schmelzrundens. Die elliptische Kante 44 der Öffnung 41 des Kanals 40 wird mit dem Laserpuls geschmolzen und es bildet sich der gewünschte, stumpfe und somit nicht schneidende, schmelzgerundete Teil 44'. Die Art, d.h. der Querschnitt des schmelzgerundeten Teils 44' der Kante 44 kann, genauso wie seine Grösse mit der Energie, dem Durchmesser des Arbeits- bzw. Wirkungsbereichs 45' und beispielsweise den Schutzgasbedingungen, beeinflusst und verändert werden. Das Schmelzrunden der Kante 44 der Hohlnadel 4, die auf einer Transporteinrichtung (nicht gezeichnet) montiert wäre, könnte auch mit einem kontinuierlich arbeitenden Laser bei entsprechender Verweilzeit des zu rundenen Teils 44' der Kante 44 erreicht und beeinflusst werden.

## Patentansprüche

1. Hohlnadel (1) zur medizinischen Verwendung, mit Kanal (10), der im Bereich der Spitze (1') der Nadel (1) eine Öffnung (11) aufweist, deren Kante (14) mindestens in dem von der Spitze (1') betrachtet entfernteren, konvergierenden Teil (14') gerundet ist, dadurch gekennzeichnet, dass die Rundung schmelzgerundet ist.

2. Hohlnadel (1) nach Anspruch 1, bei der die Rundung mit einem Laserstrahl schmelzgerundet ist.

3. Hohlnadel (1) nach Anspruch 1 oder 2, mit Abschrägung (12) im Bereich der Spitze (1'), in welche die Öffnung (11) mündet.

4. Hohlnadel (1) nach Anspruch 1, 2 oder 3, mit Biegung von Nadel (1) und Kanal (10) im Bereich der Spitze (1').

5. Hohlnadel (1) nach Anspruch 4, mit einer Biegung, deren äusserer Biegeradius nicht mehr als dem dreifachen Durchmesser der Hohlnadel (1) entspricht.

6. Hohlnadel (2) nach einem der Ansprüche 1 bis 5, mit facettengeschliffener Spitze (2', 2'', 2''').

7. Hohlnadel (1) nach einem der Ansprüche 1 bis 6 mit geschliffener Abschrägung, deren Mittelebene mit der Achse der Hohlnadel (1) einen Winkel (α) im Bereich von -10° bis +40° bildet.

8. Hohlnadel (1) nach einem der Ansprüche 1 bis 7 mit einem Aussendurchmesser im Bereich von 0,2 bis 0,4 mm.

9. Verfahren zum Herstellen von Hohlnadeln (4) nach einem der Ansprüche 1 bis 8, bei welchem die Kante (44) des Kanals (40) mit Laserlicht (45) eines gepulst oder kontinuierlich betriebenen Lasers schmelzgerundet wird, wobei Laserlicht (45') auf den zu schmelzenden Bereich (44') der Kante (44) gerichtet wird.

10. Verfahren nach Anspruch 9, bei dem ein gepulst betriebener Laser verwendet wird, wobei der Durchmesser des Laserstrahls (45) im Wirkbereich (45') eine Grösse im Bereich von 0.5 bis 3 mm und die Gesamtenergie eines Pulses im Bereich von 0,02 bis 20 Joule liegen, wobei der Durchmesser des Laserstrahl (45) im Wirkungsbereich (45') etwa dem dreifachen Durchmesser der zu bearbeitenden Hohlnadel (4) entspricht.

11. Verfahren nach Anspruch 9 oder 10, bei welchem das Schmelzrunden der Kante (44) des Kanals (40) unter Schutzgas erfolgt.

12. Verfahren nach einem der Ansprüche 9 bis 11, bei welchem Grösse und Form des Arbeitsflecks (45') des Laserstrahls (45) der zu rundenden Zone (44') der Kante (44) des Kanals (40) der Hohlnadel (4) angepasst ist.

## Claims

1. Hollow needle (1) for medical applications with a passage (10) which has an opening (11) in the region of the tip (1') of the needle (1) with the edge (14) of the opening being rounded at least in the more remote convergent part (14') when viewed from the tip (1'), characterized in that the rounded edge is melt rounded.

2. Hollow needle (1) in accordance with claim 1 in which the rounded edge is melt rounded by a laser beam.

3. Hollow needle (1) in accordance with claim 1 or 2 with a bevel (12) in the vicinity of the tip (1') into which the opening (11) opens.

4. Hollow needle (1) in accordance with claim 1, 2 or 3 with bending of the needle (1) and the passage (10) in the vicinity of the tip (1').

5. Hollow needle (1) in accordance with claim 4 with a bend whose outer bending radius corresponds to no more than three times the diameter of the hollow needle (1).

6. Hollow needle (2) in accordance with one of the claims 1 to 5 with facette ground tip (2', 2'', 2''').

7. Hollow needle (1) in accordance with one of the claims 1 to 6 with a ground bevel whose midplane forms an angle (α) with the axis of the hollow needle in the range from -10° to +40°.

8. Hollow needle (1) in accordance with one of the claims 1 to 7 with an external diameter in the range from 0.2 to 0.4 mm.

9. Method for manufacturing hollow needles (4) in accordance with one of the claims 1 to 8 in which the edge (44) of the passage (40) is melt rounded with the light (45') of a pulsed or continuous wave laser, with laser light (45') being directed onto the region (44') of the edge (44) to be melted.

10. Method in accordance with claim 9 in which a pulsed laser is used, with the diameter of the laser beam (45) in the effective region (45') having a value in the range from 0.5 to 3 mm and the total energy of a pulse being in the range from 0.02 to 20 joules and the diameter of the laser beam (45) in the effective region (45') corresponding to about three times the diameter of the hollow needle (4) to be processed.

11. Method in accordance with claim 9 or 10 in which the melt rounding of the edge (44) of the passage (40) takes place in a protective gas atmosphere.

12. Method in accordance with one of the claims 9 to 11 in which the size and form of the working spot (45') of the laser beam (45) is adapted to the zone (44') to be rounded of the edge (44) of the passage (40) of the hollow needle (4).

## Revendications

1. Aiguille creuse (1) à usage médical, comprenant un canal (10) qui, dans la zone de la pointe (1') de l'aiguille (1), comporte un orifice (11) dont l'arête (14) est arrondie au moins dans la partie convergente (14') qui, vue de la pointe (1'), est la plus éloignée, caractérisée en ce que l'arrondi est réalisé par fusion.

2. Aiguille creuse (1) selon la revendication 1, dans laquelle l'arrondi est réalisé par fusion avec un faisceau laser.

3. Aiguille creuse (1) selon la revendication 1 ou 2, comprenant, dans la zone de la pointe (1'), un biseau (12) dans lequel débouche l'orifice (11).

4. Aiguille creuse (1) selon la revendication 1, 2 ou 3, présentant une courbure de l'aiguille (1) et du canal (10) dans la zone de la pointe (1').

5. Aiguille creuse (1) selon la revendication 4, présentant une courbure dont le rayon extérieur de courbure correspond au plus à trois fois le diamètre de l'aiguille creuse (1).

6. Aiguille creuse (2) selon l'une des revendications 1 à 5, présentant une pointe taillée à facettes (2', 2'', 2''')`

7. Aiguille creuse (1) selon l'une des revendications 1 à 6, présentant un biseau taillé dont le plan médian forme, avec l'axe de l'aiguille creuse (1) , un angle α compris entre - 10° et + 40°.

8. Aiguille creuse (1) selon l'une des revendications 1 à 7, présentant un diamètre extérieur compris entre 0,2 et 0,4 mm.

9. Procédé pour la fabrication d'aiguilles creuses (4) selon l'une des revendications 1 à 8, dans lequel l'arête (44) du canal (40) est arrondie par fusion avec la lumière laser (45) d'un laser en régime pulsé ou continu, la lumière laser (45') étant dirigée sur la zone à fondre (44') de l'arête (44).

10. Procédé selon la revendication 9, dans lequel un laser à régime pulsé est mis en oeuvre, le diamètre du faisceau laser (45) présentant une dimension de 0,5 à 3 mm dans la zone d'action (45') et l'énergie totale d'une impulsion étant comprise entre 0,02 et 20 joules, le diamètre du faisceau laser (45) dans la zone d'action (45') correspondant sensiblement à trois fois le diamètre de l'aiguille creuse (4) que l'on veut traiter.

11. Procédé selon la revendication 9 ou 10, dans lequel l'arrondissage par fusion de l'arête (44) du canal (40) s'effectue sous gaz protecteur.

12. Procédé selon l'une des revendications 9 à 11, dans lequel la taille et la forme de la tache de travail (45') du faisceau laser (45) sont adaptées à la zone à arrondir (44') de l'arête (44) du canal (40) de l'aiguille creuse (4).
